# EUROPÄISCHE PATENTANMELDUNG

(11) **EP 4 649 970 A1**
(43) Veröffentlichungstag der Anmeldung: **19.11.2025**
(21) Anmeldenummer: 25174161.7
(22) Anmeldetag: 05.05.2025
(51) Int. Cl.: A61M 1/14, F16B 5/02, F16B 25/00

(54) **BAUTEILANORDNUNG FÜR EIN MEDIZINISCHES GERÄT**

(30) Priorität: 13.05.2024 DE 202024102439 U
(71) Anmelder: B. Braun Avitum AG, 34212 Melsungen (DE)
(72) Erfinder: Knierim, Michael, 37242 Bad Sooden-Allendorf (DE); Schwendich, Wilhelm, 36100 Petersberg (DE); Janik, Waldemar, 34212 Melsungen (DE)
(74) Vertreter: Patentanwälte Ruff, Wilhelm, Beier, Dauster & Partner mbB

(57) **Zusammenfassung**

Die Erfindung betrifft eine Bauteilanordnung für ein medizinisches Gerät, insbesondere ein Dialysegerät, aufweisend ein erstes Bauteil mit einer Vorderseite, einer Rückseite und einer Aussparung, die zwischen der Vorderseite und der Rückseite erstreckt ist, ein zweites Bauteil, das einen Schraubdom mit einem längserstreckten Domschaft und einer Bohrung aufweist, wobei das zweite Bauteil an der Rückseite des ersten Bauteils angeordnet ist und der Domschaft durch die Aussparung hindurch axial über die Vorderseite des ersten Bauteils ragt, eine Buchse, die über ein durch die Aussparung ragendes Stirnende des axial auf denselben aufgeschoben ist und auf der Vorderseite des ersten Bauteils axial abgestützt ist, und eine mit einem Schraubenschaft, der ein Gewinde aufweist, und mit einem Schraubenkopf, wobei der Schraubenschaft in die Bohrung eingeschraubt ist und der Schraubenkopf axial auf der Buchse abgestützt ist, wodurch das erste Bauteil mit dem zweiten Bauteil verschraubt ist.

## Beschreibung

Die Erfindung betrifft eine Bauteilanordnung für ein medizinisches Gerät, insbesondere ein Dialysegerät.

Medizinische Geräte, wie beispielsweise Dialysegeräte, weisen in der Regel ein aus mehreren Bauteilen zusammengefügtes Gehäuse auf, wobei als Fügeverbindungen zwischen den Bauteilen oftmals Schraubverbindungen vorgesehen sind. Bei aus Kunststoff gefertigten Bauteilen finden üblicherweise sogenannte Kunststoff-Direktverschraubungen Anwendung. Solche Schraubverbindungen umfassen einen Schraubdom, in welchen eine Schraube mit selbstfurchendem Gewinde eingedreht ist.

Aufgabe der Erfindung ist es, eine Bauteilanordnung für ein medizinisches Gerät bereitzustellen, die Vorteile gegenüber dem Stand der Technik bietet.

Diese Aufgabe wird durch das Bereitstellen einer Bauteilanordnung mit den Merkmalen des Anspruchs 1 gelöst. Vorteilhafte Weiterbildungen sind in den Unteransprüchen angegeben. Der Wortlaut der Ansprüche wird durch Bezugnahme zum Gegenstand der Beschreibung gemacht.

Die erfindungsgemäße Bauteilanordnung weist ein erstes Bauteil, ein zweites Bauteil, eine Buchse und eine Schraube auf. Das erste Bauteil weist eine Vorderseite, eine Rückseite und eine Aussparung auf. Die Aussparung ist zwischen der Vorderseite und der Rückseite erstreckt. Das zweite Bauteil ist vorzugsweise aus einem Kunststoffmaterial spritzgegossen. Das zweite Bauteil weist einen Schraubdom mit einem längserstreckten Domschaft und einer Bohrung auf. Das zweite Bauteil ist wenigstens abschnittsweise an der Rückseite des ersten Bauteils angeordnet und der Domschaft ragt ausgehend von der Rückseite axial durch die Aussparung über die Vorderseite des ersten Bauteils. Die Buchse ist über ein durch die Aussparung ragendes Stirnende des Domschafts axial auf den Domschaft aufgeschoben und axial auf der Vorderseite des ersten Bauteils abgestützt. Die Schraube weist einen Schraubenschaft mit einem Gewinde und einen Schraubenkopf auf. Der Schraubenschaft ist in die Bohrung eingeschraubt und der Schraubenkopf ist axial auf der Buchse abgestützt, wodurch das erste Bauteil mit dem zweiten Bauteil verschraubt ist. Die erfindungsgemäße Bauteilanordnung bietet unterschiedliche Vorteile gegenüber dem Stand der Technik. Insbesondere können eine vereinfachte geometrische Gestaltung des ersten Bauteils und/oder des zweiten Bauteils und damit einhergehend eine vereinfachte Fertigung erreicht werden. Zudem ergibt sich eine vereinfachte Montierbarkeit. Die genannten Vorteile werden in erster Linie durch die axial zwischen dem Schraubenkopf und der Vorderseite des ersten Bauteils angeordnete Buchse erreicht. Die Buchse dient hierbei als Abstandhalter sowie Kraftübertragungselement. Zudem hat sich gezeigt, dass die erfindungsgemäße Bauteilanordnung eine im Vergleich zu etablierten Gestaltungsregeln schlankere oder auch dünnere Ausführung des Domschafts erlaubt. Die mechanischen Eigenschaften des Schraubendoms und damit der Schraubbefestigung insgesamt werden hierdurch nicht beeinträchtigt. Sofern das zweite Bauteil aus einem Kunststoffmaterial spritzgegossen ist, können durch die schlankere Gestaltung des Schraubendoms, im Speziellen des Domschafts, Einfallstellen an dem zweiten Bauteil vermieden werden. Bei einer bevorzugten Ausgestaltung ist auch das erste Bauteil aus einem Kunststoffmaterial gefertigt, im Speziellen spritzgegossen. Bei einer Ausgestaltung handelt es sich bei dem ersten Bauteil und bei dem zweiten Bauteil jeweils um ein Gehäusebauteil des medizinischen Geräts. Bei einer weiteren Ausgestaltung ist das erste Bauteil ein Gehäusebauteil des medizinischen Geräts und das zweite Bauteil ist einer an dem Gehäuse befestigten Baugruppe zugeordnet, beispielsweise einer Kommunikationsbaugruppe (COMM-Modul). Bei einer Ausgestaltung ist die Buchse aus Metall gefertigt. Bei einer weiteren Ausgestaltung ist die Buchse aus einem Kunststoffmaterial gefertigt.

In Ausgestaltung der Erfindung ragt die Buchse über das Stirnende des Domschafts axial hinaus, wodurch zwischen dem axial auf der Buchse abgestützten Schraubenkopf und dem Stirnende des Domschafts ein Axialspalt gebildet ist. Der Axialspalt gewährleistet einen vorteilhaften Kraftfluss über die Buchse. Dabei werden der Schraubenschaft und der Schraubdom axial auf Zug beansprucht und der Schraubenkopf drückt axial auf die Buchse, die wiederum axial auf der Vorderseite des ersten Bauteils abgestützt ist. Hierdurch wird das zweite Bauteil axial gegen die Rückseite des ersten Bauteils gezogen/gepresst.

In weiterer Ausgestaltung der Erfindung ist die Buchse auf den Domschaft aufgepresst, wodurch der Domschaft beim Einschrauben des Schraubenschafts in die Bohrung mittels der Buchse radial nach außen abgestützt und gegen ein Platzen geschützt ist. Bei dieser Ausgestaltung der Erfindung sind ein Innendurchmesser der Buchse und ein Außendurchmesser des Domschafts folglich derart aufeinander abgestimmt, dass der Außendurchmesser des Domschafts sich radial nach außen an dem Innendurchmesser der Buchse abstützt. Diese radiale Abstützung liegt wenigstens in vollständig ausgebildetem Zustand der Schraubverbindung vor.

In weiterer Ausgestaltung der Erfindung ist das zweite Bauteil aus einem Kunststoffmaterial gefertigt, insbesondere spritzgegossen.

In weiterer Ausgestaltung der Erfindung ist das Gewinde selbstfurchend. Selbstfurchend meint, dass das Gewinde der Schraube beim Einschrauben in den Domschaft sein Gegengewinde selbst ausbildet, im Speziellen furcht und/oder schneidet. Das Gegenwinde muss daher nicht in einem gesonderten Fertigungsschritt in den Schraubendom eingebracht werden. Diese Ausgestaltung ist besonders dann vorteilhaft, wenn das zweite Bauteil aus einem/dem Kunststoffmaterial gefertigt ist.

In weiterer Ausgestaltung der Erfindung weist der Domschaft im Vergleich zu einer normgemäßen Gestaltung, insbesondere nach einer Norm und/oder nach einem Konstruktionshinweis eines Schraubenherstellers, einen dünneren Außendurchmesser auf. Durch den dünneren Außendurchmesser des Domschafts kann Material eingespart werden. Die Materialeinsparung geht mit einer vereinfachten Fertigung des spritzgegossenen zweiten Bauteils einher und hilft insbesondere, Einfallstellen im Bereich des Schraubdoms zu vermeiden. Solche Einfallstellen können die mechanischen Eigenschaften des zweiten Bauteils beeinträchtigen. Auch der optische Eindruck kann leiden. Beides kann dazu führen, dass Ausschuss entsteht. Durch die dünnere Gestaltung des Domschafts und dessen Zusammenwirken mit den übrigen Komponenten der erfindungsgemäßen Bauteilanordnung wird alldem auf überraschend einfache, aber sehr wirkungsvolle Weise entgegengewirkt.

In weiterer Ausgestaltung der Erfindung ist die Schraube mit einer Unterlegscheibe versehen, die form- und/oder kraftschlüssig mit einer Unterseite des Schraubenkopfs verbunden ist. Die Unterlegscheibe ist folglich axial zwischen dem Schraubenkopf und der Buchse angeordnet. Durch die Abmessungen und/oder Materialeigenschaften der Unterlegescheibe können die Eigenschaften der Schraubverbindung positiv beeinflusst werden.

In weiterer Ausgestaltung der Erfindung sind die Buchse und die Schraube verliersicher aneinander gehalten. Mit anderen Worten: Zwischen der Schraube und der Buchse ist eine Verliersicherung ausgebildet. Hierdurch wird eine vereinfachte Montierbarkeit der Bauteilanordnung erreicht. Durch die Verliersicherung können die Schraube und die Buchse gemeinsam und einhändig montiert werden. Hierdurch wird zum einen Zeit eingespart. Zum anderen wird vermieden, dass die Buchse bei der Montage aus Versehen weggelassen wird. Hierdurch werden Montagefehler und Folgeschäden vermieden. Bei einer Ausgestaltung umfasst die Verliersicherung eine zwischen der Schraube und der Buchse ausgebildete formschlüssige Verbindung. Alternativ oder zusätzlich umfasst die Verliersicherung eine kraftschlüssige und/oder stoffschlüssige Verbindung.

In weiterer Ausgestaltung der Erfindung weist die Buchse einen Innendurchmesser auf, der auf einen Außendurchmesser des Schraubenschafts axial aufgeschoben ist, wobei zwischen dem Innendurchmesser der Buchse und dem Außendurchmesser des Schraubenschafts eine Presspassung ausgebildet ist. Die Presspassung bildet eine reibschlüssige Verbindung zwischen der Schraube und der Buchse und damit die besagte Verliersicherung aus. Die reibschlüssige Verbindung wirkt axial und in Umfangsrichtung. Radial liegt ein Formschluss zwischen dem Innen- und dem Außendurchmesser vor.

In weiterer Ausgestaltung der Erfindung weist die Buchse ein Innengewinde auf, das mit einem Außengewinde des Schraubenschafts verschraubt ist. Bei dieser Ausgestaltung ist die Verliersicherung eine Schraubverbindung zwischen der Schraube und der Buchse. Bei einer Ausgestaltung ist das mit dem Innengewinde der Buchse verschraubte Außengewinde das erwähnte selbstfurchende Gewinde. Bei einer weiteren Ausgestaltung ist das Außengewinde ein hiervon separates Gewinde mit unterschiedlicher Gestaltung. Beispielsweise kann das Außengewinde relativ zu dem selbstfurchenden Gewinde axial in Richtung des Schraubenkopfs versetzt angeordnet sein. Bei einer Ausgestaltung ist das Innengewinde der Buchse mittels des Außengewindes der Schraube gefurcht und/oder geschnitten. Bei einer weiteren Ausgestaltung ist das Innengewinde der Buchse bereits vor dem Zusammenfügen der Buchse mit der Schraube vorhanden und/oder vorgeschnitten.

In weiterer Ausgestaltung der Erfindung weist die Buchse eine Rastgeometrie auf, die mit einer komplementären Rastgeometrie der Schraube verrastet ist. Bei dieser Ausgestaltung ist die Verliersicherung eine zwischen der Rastgeometrie der Buchse und der komplementären Rastgeometrie der Schraube ausgebildete Rastverbindung. Bei einer Ausgestaltung ist die komplementäre Rastgeometrie an dem Schraubenschaft ausgebildet. Bei einer weiteren Ausgestaltung ist die komplementäre Rastgeometrie an dem Schraubenkopf ausgebildet. Bei einer weiteren Ausgestaltung ist die komplementäre Rastgeometrie abschnittsweise an dem Schraubenschaft und abschnittsweise an dem Schraubenkopf ausgebildet. Bei einer Ausgestaltung ist diese Rastverbindung lösbar. Bei einer weiteren Ausgestaltung ist die Rastverbindung unlösbar.

Die Erfindung betrifft zudem ein medizinisches Gerät mit einer Bauteilanordnung gemäß der vorhergehenden Beschreibung. Im Speziellen handelt es sich bei dem medizinischen Gerät um ein Dialysegerät zur extrakorporalen Blutbehandlung.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivansicht eine Ausführungsform eines erfindungsgemäßen medizinischen Geräts in Form eines Dialysegeräts mit einem Gehäuse, das eine Ausführungsform einer erfindungsgemäßen Bauteilanordnung aufweist,
- Fig. 2: in schematischer Perspektivansicht die erwähnte Bauteilanordnung des medizinischen Geräts nach Fig. 1,
- Fig. 3: eine längsgeschnittene Explosionsdarstellung der Bauteilanordnung nach Fig. 2 im Bereich eines Schraubdoms,
- Fig. 4: der Bereich nach Fig. 3 in einem schematischen Längsschnitt,
- Fig. 5: ein schematischer Längsschnitt einer weiteren Ausführungsform einer erfindungsgemäßen Bauteilanordnung im Bereich eines Schraubdoms mit reibschlüssiger Verliersicherung zwischen einer Schraube und einer Buchse der Bauteilanordnung,
- Fig. 6: eine Variante der Bauteilanordnung nach Fig. 5,
- Fig. 7: in einer den Fig. 5 und 6 entsprechenden Darstellungsweise eine weitere Ausführungsform einer erfindungsgemäßen Bauteilanordnung mit einer formschlüssigen Verliersicherung zwischen Schraube und Buchse,
- Fig. 8: eine Variante der Bauteilanordnung nach Fig. 7,
- Fig. 9: in einer den Fig. 5 bis 8 entsprechenden Darstellungsweise eine weitere Ausführungsform einer erfindungsgemäßen Bauteilanordnung mit einer durch eine Rastverbindung gebildete Verliersicherung zwischen Schraube und Buchse und
- Fig. 10, 11: jeweils in einer den Fig. 5 bis 9 entsprechenden Darstellungsweise Varianten der Bauteilanordnung nach Fig. 9.

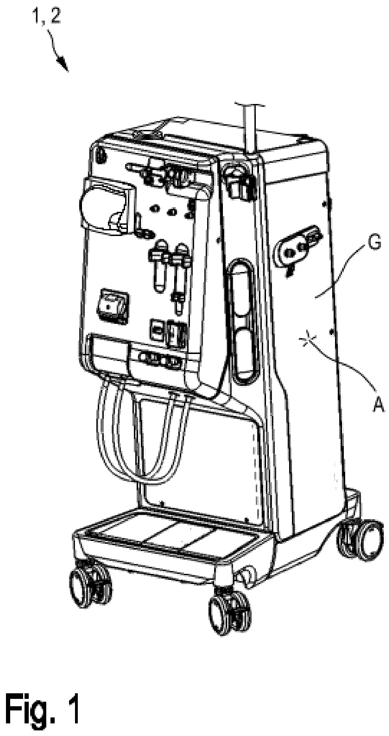

Gemäß Fig. 1 ist ein medizinisches Gerät 1 in Form eines Dialysegeräts 2 zur Verwendung bei einer extrakorporalen Blutbehandlung vorgesehen. Das Dialysegerät 2 weist eine der Fachperson grundsätzlich bekannte Funktion auf, so dass auf diesbezügliche Merkmale des Dialysegeräts 2 nicht weiter eingegangen werden braucht.

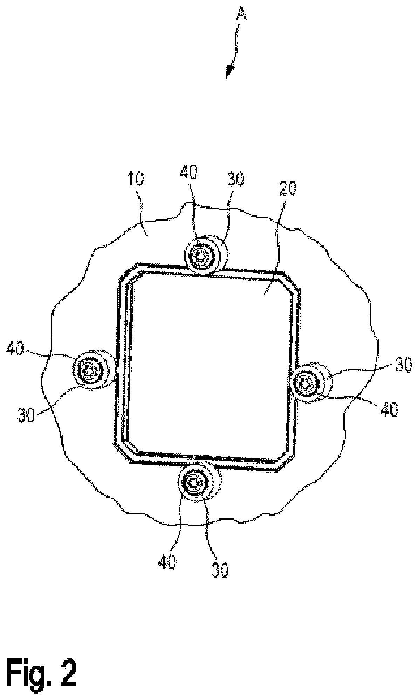

Das Dialysegerät 2 weist ein Gehäuse G auf. Das Gehäuse G umfasst eine Bauteilanordnung A (in Fig. 1 nicht ersichtlich), wie sie im Detail in den Fig. 2 bis 4 gezeigt ist.

Die Bauteilanordnung A weist ein erstes Bauteil 10, ein zweites Bauteil 20, eine Buchse 30 und eine Schraube 40 auf.

Das erste Bauteil 10 und das zweite Bauteil 20 sind auf noch näher beschriebene Weise miteinander verschraubt. Dabei sind bei der gezeigten Ausführungsform insgesamt vier Schrauben und vier Buchsen vorhanden (siehe insbesondere Fig. 2). Die gezeigte Anzahl ist als rein exemplarisch zu verstehen. Nachfolgend werden die weiteren Merkmale der Bauteilanordnung A deshalb unter Bezugnahme auf eine/die Schraube 30 und eine/die Buchse 40 erläutert. Für die weiteren in Fig. 2 gezeigten Schrauben und Buchsen sowie deren Zusammenwirken gilt grundsätzlich das im Hinblick auf die Schraube 30 und die Buchse 40 Gesagte sinngemäß.

Das erste Bauteil 10 weist eine Vorderseite 11, eine Rückseite 12 und eine Aussparung 13 auf. Die Aussparung 13 ist von der Vorderseite 11 bis auf die Rückseite 12 durchgängig erstreckt.

Das zweite Bauteil 20 ist bei der gezeigten Ausführungsform aus einem Kunststoffmaterial K gefertigt. Die Fertigung erfolgt vorliegend per Spritzguss. Es sind auch andere Fertigungsweisen denkbar und möglich, beispielsweise eine additive Fertigung, im Speziellen per 3D-Druck. Das zweite Bauteil 20 weist einen Schraubdom 21 mit einem längserstreckten Domschaft 22 und einer Bohrung 23 auf. Das zweite Bauteil 20 ist an der Rückseite 12 des ersten Bauteils 10 angeordnet. Dabei ragt der Schraubdom 21 axial durch die Aussparung 13. Im Speziellen ragt der Domschaft 22 ausgehend von der Rückseite 12 durch die Aussparung 13 über die Vorderseite 11 des ersten Bauteils. Dabei ist ein Stirnende 24 des Schraubdoms 21 in einem nicht näher bezeichneten axialen Abstand oberhalb der Vorderseite 11 positioniert.

Die Buchse 30 ist über das Stirnende 24 des Domschafts 22 axial auf den Domschaft 22 aufgeschoben und stützt sich axial auf der Vorderseite 11 des ersten Bauteils 10 ab. Vorliegend weist die Buchse ein erstes Stirnende 31, ein zweites Stirnende 32, einen Innenumfang 33 und einen Außenumfang 34 auf. Die Buchse 30 ist axial zwischen dem ersten Stirnende 31 und dem zweiten Stirnende 32 längserstreckt. Radial ist die Buchse 30 zwischen dem Innenumfang 33 und dem Außenumfang 34 erstreckt.

Sowohl die Buchse 30 als auch der Schraubdom 21 weisen vorliegend jeweils eine zylindrische Gestalt auf.

Die Schraube 40 weist einen Schraubenschaft 41 mit einem Gewinde 42 und einen Schraubenkopf 43 auf. Bei der gezeigten Ausführungsform ist das Gewinde 42 selbstfurchend. Der Schraubenschaft 41 ist in die Bohrung 23 des Schraubdoms 21 eingeschraubt. Beim Einschrauben der Schraube 40 schneidet das selbstfurchende Gewinde 42 gleichsam ihr eigenes Gegengewinde in die Bohrung 23. Bei Ausgestaltungen mit nicht selbstfurchendem Gewinde ist das Gegengewinde vorgeschnitten. Der Schraubenkopf 43 ist axial auf der Buchse 30 abgestützt. Genauer ist der Schraubenkopf 43 axial auf dem ersten Stirnende 31 der Buchse 30 abgestützt, deren zweites Stirnende 32 ist auf der Vorderseite 11 des ersten Bauteils 10 axial abgestützt.

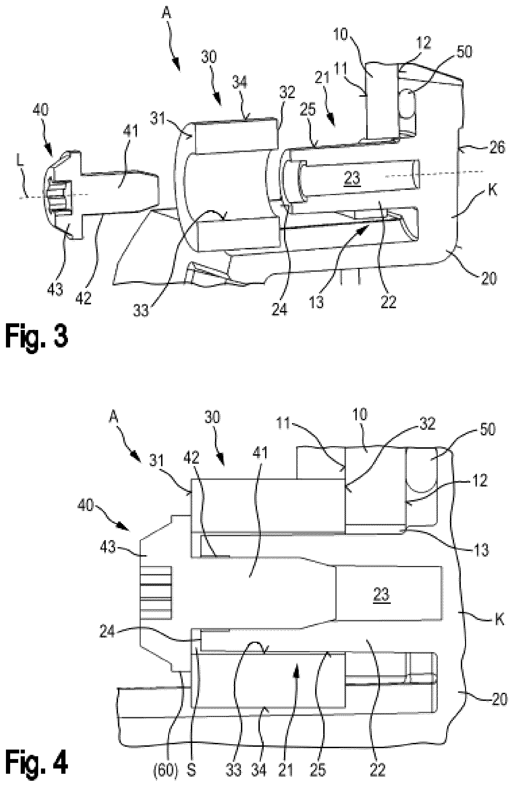

Die in Fig. 4 gezeigte zwischen dem Schraubdom 21, der Schraube 40 und den weiteren Bauteilen der Bauteilanordnung A gebildete Schraubverbindung kann auch als Kunststoffdirektverschraubung bezeichnet werden. Anstelle der gezeigten Kunststoffdirektverschraubung ist bei weiteren Ausgestaltungen ein metrisches oder sonstiges Gewinde vorgesehen.

Das erste Stirnende 31 der Buchse 30 ragt axial über das Stirnende 24 des Domschafts 22 hinaus (siehe Fig. 4). Hierdurch ist zwischen dem Schraubenkopf 43 und dem Stirnende 24 des Domschafts 22 ein Axialspalt S ausgebildet.

Anders als Fig. 4 zunächst vermuten lässt, ist ein Außenumfang 25 des Domschafts 22 radial nach außen an dem Innenumfang 33 der Buchse abgestützt. Durch diese radiale Abstützung des Domschafts 22 mittels der Buchse 30 wird vermieden, dass der Domschaft 22 beim Anziehen der Schraubverbindung "platzt", d. h. mechanisch überbeansprucht wird und versagt.

Weiter weist der Domschaft 22 bei der gezeigten Ausführungsform einen im Vergleich zu einer normgemäßen Gestaltung dünneren Außendurchmesser (ohne Bezugszeichen) auf. Durch den reduzierten Außendurchmesser des Domschafts 22 wird eine Anhäufung des Kunststoffmaterials K im Bereich des Schraubdoms 21 vermieden. Solche Materialanhäufungen können bei der Fertigung mittels Spritzgießens zu Einfallstellen führen, die sich insbesondere an einer Rückseite 26 des zweiten Bauteils 20 ausbilden können. Solche Einfallstellen sind in unterschiedlicher Hinsicht nachteilig. Durch die dünne Gestaltung des Schraubdoms 21 wird solchen Nachteilen entgegengewirkt.

Im Übrigen weist die Bauteilanordnung A bei der gezeigten Ausführungsform ein Dichtelement 50 auf, das als optional zu verstehen ist. Das Dichtelement 50 ist zwischen der Rückseite 12 des ersten Bauteils 10 und einer nicht näher bezeichneten Vorderseite des zweiten Bauteils 20 angeordnet. Beim Anziehen der Schraubverbindung wird das Dichtelement 50 komprimiert und/oder gequetscht.

Weiter weist die Bauteilanordnung A vorliegend eine optionale Unterlegscheibe 60 auf. Die Unterlegscheibe 60 ist in Fig. 4 einstückig mit dem Schraubenkopf 43 gezeigt, was allein zeichnerische Gründe hat. Die Unterlegscheibe 60 dient einer verbesserten Kraftübertragung zwischen Schraubenkopf 43 und Buchse 30.

Bei der in den Fig. 2 bis 4 gezeigten Ausführungsform sind die Buchse 30 und die Schraube 40 in nicht montiertem Zustand der Bauteilanordnung A räumlich voneinander getrennt. Mit anderen Worten: Die Buchse 30 und die Schraube 40 werden separat in aufeinanderfolgenden Montageschritten montiert. Dabei wird zunächst die Buchse 30 auf den Schraubdom 21 axial aufgeschoben. Hiernach wird die Schraube 40 mit dem Schraubdom 21 verschraubt.

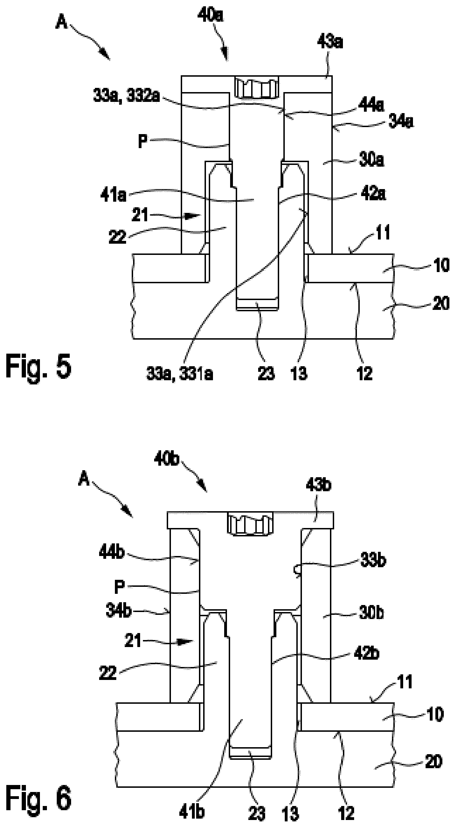

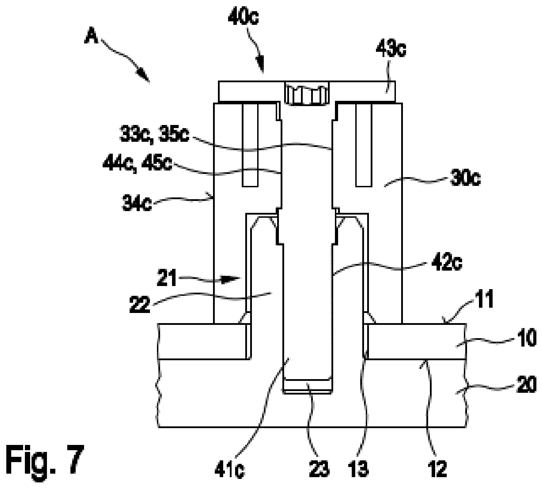

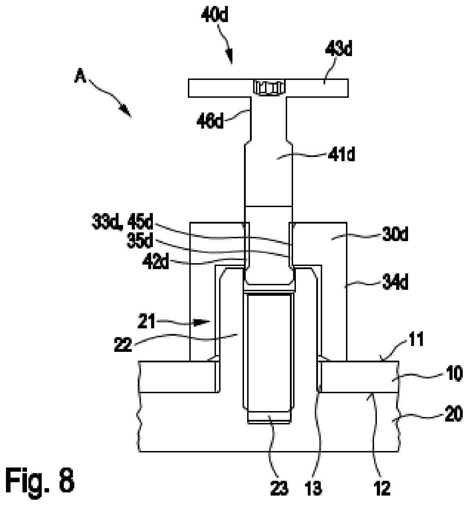

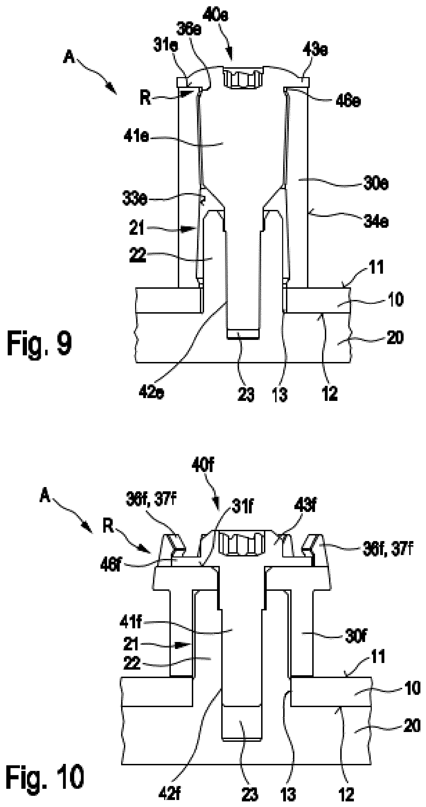

Zur Vereinfachung der Montage und zur Vermeidung von Montagefehlern können die Schraube und die Buchse verliersicher aneinander gehalten, d.h. vormontiert, sein. Die aneinander gehaltenen/vormontierten Anordnungen aus Schraube und Buchse können auch als Kombielement bezeichnet werden. Dabei sind in den Fig. 5 bis 11 weitere Bauteilanordnungen A gezeigt, die eine solche Verliersicherung zwischen Schraube und Buchse vorsehen. Die Verliersicherungen sind bei den in den Fig. 5 bis 11 gezeigten Ausführungsformen auf unterschiedliche Weise gestaltet. Bei den in den Fig. 5 und 6 gezeigten Varianten ist eine reibschlüssige Verliersicherung vorgesehen. Bei den in den Fig. 7 und 8 gezeigten Varianten dient eine Schraubverbindung zwischen Schraube und Buchse als Verliersicherung. Bei den in den Fig. 9 bis 11 gezeigten Varianten sind Schraube und Buchse zum Ausbilden der Verliersicherung miteinander verrastet. Weitere Merkmale der in den Fig. 5 bis 11 gezeigten Ausführungsformen werden nachfolgend im Detail erläutert.

Bei der in Fig. 5 gezeigten Ausführungsform weist der Innenumfang 33a der Buchse 30a einen ersten Innendurchmesser 331a und einen zweiten Innendurchmesser 332a auf. Der Innenumfang 33a ist folglich radial gestuft. Dabei ist der zweite Innendurchmesser 332a kleiner als der erste Innendurchmesser 331a und maßlich auf einen Außendurchmesser 44a des Schraubenschafts 41a abgestimmt. Im Speziellen ist eine Presspassung P zwischen dem Außendurchmesser 44a und dem zweiten Innendurchmesser 33a ausgebildet. Die Presspassung P bewirkt eine reibschlüssige Verliersicherung zwischen der Schraube 40a und der Buchse 30a.

In Fig. 6 ist eine Variante mit ebenfalls reibschlüssiger Verliersicherung gezeigt. Im Unterschied zu der Ausführungsform nach Fig. 5 ist der Innenumfang 33b der Buchse 30b nicht gestuft, sondern durchgängig gerade. Dabei richtet sich das Maß des Innenumfangs 33b in erster Linie nach dem Außendurchmesser des Domschafts 21. Die Schraube 40b weist einen gestuften Schraubenschaft 41b auf, wobei das selbstfurchende Gewinde 42b an einem radial zurückgesetzten Bereich des Schraubenschafts 41b ausgebildet ist. Die Presspassung P ist im Bereich eines erweiterten Außendurchmessers 44b ausgebildet.

Bei der in Fig. 7 gezeigten Ausführungsform ist der Innenumfang 33c der Buchse 30c mit einem Innengewinde 35c versehen. Der Außenumfang 44c des Schraubenschafts 41c weist ein (weiteres) Außengewinde 45c auf. Das Außengewinde 45c ist mit dem Innengewinde 35c verschraubt. Hierdurch sind die Buchse 30c und die Schraube 40c verliersicher aneinander gehalten. Dabei ist die besagte Schraubverbindung zwischen dem Außengewinde 45c und dem Innengewinde 35c sowohl bei der Montage als auch nach der Montage der Bauteilanordnung A im Eingriff. Durch die (zusätzliche) Verschraubung der Schraube 40c mit der Buchse 30c kann möglicherweise ein Anziehen oder Lösen der Schraubverbindung zwischen dem selbstfurchenden Gewinde 42c und der Bohrung 23 erschwert werden. Das Innengewinde 35c ist vorliegend durch das Außengewinde 45c gefurcht und/oder geschnitten. Bei einer weiteren Ausgestaltung kann das Innengewinde aber auch vorgeschnitten und insoweit bereits vor einem Verschrauben von Schraube und Buchse vorhanden sein.

Um dem entgegenzuwirken, ist bei der in Fig. 8 gezeigten Variante eine alternative Art der Verliersicherung zwischen der Schraube 40d und der Buchse 30d. Bei dieser Art der Verliersicherung sind/bleiben die Schraube 40d und die Buchse 30d in Umfangsrichtung zu einander freigängig. Hierbei bildet das selbstfurchende Gewinde 42d gleichzeitig das Außengewinde 45d zum Verschrauben mit dem Innengewinde 35d der Buchse 30d. Das selbstfurchend Gewinde 42d bzw. das Außengewinde 45d erstreckt sich axial nicht vollständig bis zu dem Schraubenkopf 43d. Stattdessen weist der Schraubenschaft 41d einen radialen Rücksprung 46d auf, der axial unmittelbar an den Schraubenkopf 43d angrenzt. In vollständig montiertem Zustand der Bauteilanordnung A ist der axiale Rücksprung 46d axial auf Höhe des Innengewindes 35d der Buchse 30d angeordnet, wobei keine Wirkverbindung zwischen dem radialen Rücksprung 46d und dem Innengewinde 35d besteht. Hierdurch ist gewährleistet, dass die Schraube 40d relativ zur Buchse 30d freigängig bleibt und nicht etwa durch eine (zusätzliche) Verschraubung mit der Buchse 30d in ihrer Beweglichkeit beeinträchtigt wird. Mit anderen Worten: Im Unterschied zu der Ausführungsform nach Fig. 7, kann die Schraube 40d sich innerhalb der Buchse 30d drehen, insbesondere infolge des in Relation dünneren Schraubenschaft im oberen Bereich.

Bei der Ausführungsform nach Fig. 9 weist die Buchse 30e eine Rastgeometrie 36e auf und die Schraube 40e weist eine komplementäre Rastgeometrie 46e auf.

Die Rastgeometrie 36e ist im Bereich des ersten Stirnendes 31e der Buchse 30e angeordnet. Im Speziellen ist die Rastgeometrie 36e ein radialer Vorsprung an dem Innenumfang 33e.

Die komplementäre Rastgeometrie 46e ist unmittelbar unterhalb des Schraubenkopfs 43e an dem Schraubenschaft 41e angeordnet und vorliegend ein radialer Rücksprung. Sowohl die Rastgeometrie 36e als auch die komplementäre Rastgeometrie 46e sind in Umfangsrichtung durchgängig umlaufend, so dass die Rastverbindung R auch als Ringverrastung bezeichnet werden kann. Denkbar und möglich ist aber auch eine nicht durchgängige umlaufende Gestaltung der Rastgeometrie 36e und/oder der komplementären Rastgeometrie 46e.

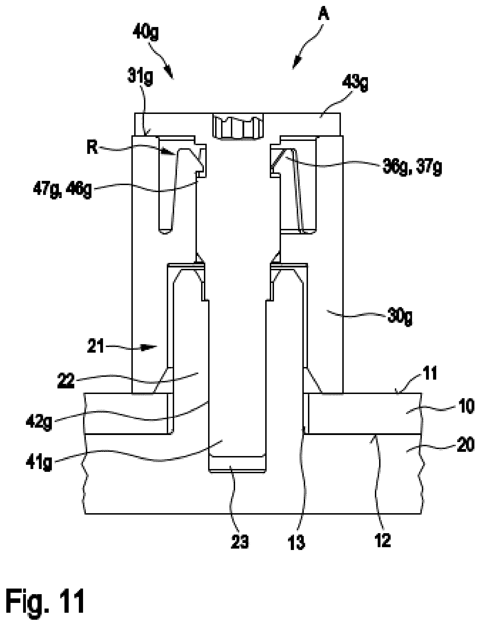

Hierzu im Unterschied ist bei den Varianten nach Fig. 10 und 11 eine jeweils an diskreten Stellen des Umfangs ausgebildete Verrastung bzw. Rastverbindung R vorhanden.

Bei der in Fig. 10 gezeigten Variante ist die Rastverbindung R zwischen der Buchse 30f und dem Schraubenkopf 43f ausgebildet. Dabei weist die Buchse 30f mehrere in Umfangsrichtung zueinander versetzt angeordnete Rastelemente 37f auf, die jeweils axial von dem ersten Stirnende 31f aufragen und mit der komplementären Rastgeometrie 46f des Schraubenkopfs 43 verrastet sind. Die komplementäre Rastgeometrie 46f ist vorliegend durch eines Außenrand des Schraubenkopfs 43f gebildet. Anstelle der an diskreten Stellen ausgebildeten Rastelemente 37f kann auch ein durchgängiger Bund oder dergleichen vorhanden sein.

Hierzu im Unterschied sieht die Variante nach Fig. 11 eine Verrastung mit dem Schraubenschaft 41g vor. Die komplementäre Rastgeometrie 46g ist ein Radialbund 47g, der axial unterhalb des Schraubenkopfs 43g angeordnet ist. Die Buchse 30g weist wiederum mehrere in Umfangsrichtung zueinander versetzt angeordnete Rastelemente 37g als Rastgeometrie 36g auf. Im Unterschied zu der Variante nach Fig. 10 sind die Rastelemente 37g axial in Richtung des zweiten Stirnendes 32g von dem ersten Stirnende 31g zurückversetzt. Dabei überragt das erste Stirnende 31g die Rastelemente 37g axial.

Bei einer weiteren Ausgestaltung weist die Buchse an ihrem Innenumfang eine radial nach innen ragenden Engstelle auf. Bei einer Vormontage der Schraube mit der Buchse wandert das Gewinde der Schraube axial über die besagte Engstelle, wodurch die Schraube und die Buchse verliersicher aneinander gehalten werden.

## Patentansprüche

1. Bauteilanordnung (A) für ein medizinisches Gerät (1), insbesondere ein Dialysegerät (2), aufweisend
ein erstes Bauteil (10) mit einer Vorderseite (11), einer Rückseite (12) und einer Aussparung (13), die zwischen der Vorderseite (11) und der Rückseite (12) erstreckt ist,
ein zweites Bauteil (20), das einen Schraubdom (21) mit einem längserstreckten Domschaft (22) und einer Bohrung (23) aufweist, wobei das zweite Bauteil (20) an der Rückseite (12) des ersten Bauteils (10) angeordnet ist und der Domschaft (22) durch die Aussparung (13) hindurch axial über die Vorderseite (11) des ersten Bauteils (10) ragt,
eine Buchse (30, 30a bis 30g), die über ein durch die Aussparung (13) ragendes Stirnende (24) des Domschafts (22) axial auf denselben aufgeschoben ist und auf der Vorderseite (11) des ersten Bauteils (10) axial abgestützt ist,
und eine Schraube (40, 40a bis 40g) mit einem Schraubenschaft (41, 41a bis 41g), der ein Gewinde (42, 42a bis 42g) aufweist, und mit einem Schraubenkopf (43, 43a bis 43g), wobei der Schraubenschaft (41, 41a bis 41g) in die Bohrung (23) eingeschraubt ist und der Schraubenkopf (43, 43a bis 43g) axial auf der Buchse (30, 30a bis 30g) abgestützt ist, wodurch das erste Bauteil (10) mit dem zweiten Bauteil (20) verschraubt ist.

2. Bauteilanordnung (A) nach Anspruch 1, wobei die Buchse (30, 30a bis 30g) über das Stirnende (24) des Domschafts (20) axial hinausragt, wodurch zwischen dem axial auf der Buchse (30, 30a bis 30g) abgestützten Schraubenkopf (43, 43a bis 43g) und dem Stirnende (24) des Domschafts (22) ein Axialspalt (S) ausgebildet ist.

3. Bauteilanordnung (A) nach Anspruch 1 oder 2, wobei die Buchse (30, 30a bis 30g) auf den Domschaft (22) aufgepresst ist, wodurch der Domschaft (22) beim Einschrauben des Schraubenschafts in die Bohrung (23) mittels der Buchse (30, 30a bis 30g) radial nach außen abgestützt und gegen ein Platzen geschützt ist.

4. Bauteilanordnung (A) nach einem der vorhergehenden Ansprüche, wobei das zweite Bauteil (20) aus einem Kunststoffmaterial (K) gefertigt, insbesondere spritzgegossen, ist.

5. Bauteilanordnung (A) nach einem der vorhergehenden Ansprüche, wobei das Gewinde (42, 42a bis 42g) selbstfurchend ist.

6. Bauteilanordnung (A) nach einem der vorhergehenden Ansprüche, wobei der Domschaft (22) im Vergleich zu einer normgemäßen Gestaltung, insbesondere nach einer Norm und/oder einem Konstruktionshinweis eines Schraubenherstellers, einen dünneren Außendurchmesser aufweist.

7. Bauteilanordnung (A) nach einem der vorhergehenden Ansprüche, wobei die Schraube (40) mit einer Unterlegscheibe (60) versehen ist, die form- und/oder kraftschlüssig mit einer Unterseite des Schraubenkopfs (43) verbunden ist.

8. Bauteilanordnung (A) nach einem der vorhergehenden Ansprüche, wobei die Buchse (30a bis 30g) und die Schraube (40a bis 40g) verliersicher aneinander gehalten sind.

9. Bauteilanordnung (A) nach Anspruch 8, wobei die Buchse (30a, 30b) einen Innenumfang (33a, 33b) aufweist, der auf einen Außenumfang (44a, 44b) des Schraubenschafts (41a, 41b) axial aufgeschoben ist, und wobei zwischen dem Innenumfang (33a, 33b) und dem Außenumfang (44a, 44b) eine Presspassung (P) ausgebildet ist.

10. Bauteilanordnung (A) nach Anspruch 8 oder 9, wobei die Buchse (30c, 30d) ein Innengewinde (35c, 35d) aufweist, das mit einem Außengewinde (45c, 45d) des Schraubenschafts (41c, 41d) verschraubt ist.

11. Bauteilanordnung (A) nach einem der Ansprüche 8 bis 10, wobei die Buchse (30e, 30f, 30g) eine Rastgeometrie (36e, 36f, 36g) aufweist, die mit einer komplementären Rastgeometrie (46e, 46f, 46g) der Schraube (40e, 40f, 40g) verrastet ist, wobei die komplementäre Rastgeometrie (36e, 36f, 36g) an dem Schraubenschaft (41e, 41g) und/oder an dem Schraubenkopf (43f) ausgebildet ist.

12. Medizinisches Gerät (1), insbesondere Dialysegerät (2), mit einer Bauteilanordnung (A) nach einem der vorhergehenden Ansprüche.
